(19) Europäisches Patentamt
European Patent Office
Office européen des brevets

(11) Publication number: 0 149 269
B1

# (12) EUROPEAN PATENT SPECIFICATION

(45) Date of publication of patent specification: 14.11.90

(51) Int. Cl.$^5$: A 01 N 43/713, C 07 D 257/04, C 07 D 401/12

(21) Application number: 84201747.7

(22) Date of filing: 28.11.84

## (54) Heterocyclic herbicides.

(30) Priority: 15.12.83 GB 8333477

(43) Date of publication of application:
24.07.85 Bulletin 85/30

(45) Publication of the grant of the patent:
14.11.90 Bulletin 90/46

(84) Designated Contracting States:
AT BE CH DE FR GB IT LI LU NL SE

(56) References cited:
EP-A-0 029 183
FR-A-1 451 028
US-A-3 839 044
US-A-4 252 815

(73) Proprietor: SHELL INTERNATIONALE RESEARCH MAATSCHAPPIJ B.V.
Carel van Bylandtlaan 30
NL-2596 HR Den Haag (NL)

(72) Inventor: Mansuri, Muzammil Mansoor
43 Beechwood Avenue
Sittingbourne Kent (GB)
Inventor: McArthur, Alasdair
16 Park Road
Sittingbourne Kent (GB)

(74) Representative: Bennett, David Arthur Horder et al
4, York Road
London SE1 7NA (GB)

EP 0 149 269 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a composition for and a method of controlling undesired plant growth and to compounds for use in such a composition or method.

1-Phenyl-5-phenoxy-1H-tetrazole is disclosed in J.C.S., Perkin Trans 1. (1973) No. 5 pp. 469—70, and certain analogues bearing substituents on the phenoxy group are disclosed in Tetrahedron, Vol. 38, No. 24 (1982), pp. 3775—3781. Also, German patent application 1,251,327 discloses a process for the production of 1-aryl-5-chloro-1H-tetrazoles, together with the reaction of such compounds with phenol to produce the corresponding 5-phenoxy compounds. The compounds are stated to be useful in silver halide photographic emulsions; the 5-chloro compounds are also stated to have fungicidal and bactericidal activity in addition to their use as intermediates for the preparation of analogues having different substituents at the 5-position. There is no teaching in any of these references that the 5-phenoxy compounds have any herbicidal activity or any other useful biological activity.

Certain tetrazolylcarboxylic acid amide derivatives are disclosed in European patent specification No. 29183 as having herbicidal activity.

It has now been found that certain 1-aryl-5-substituted-1H-tetrazoles have useful herbicidal properties, and accordingly the present invention provides a herbicidal composition which comprises at least one carrier which is a surface active agent, and, as active ingredient, a substituted tetrazole of the formula:—

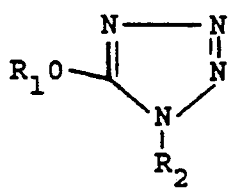

I

wherein $R_1$ represents an alkyl, cycloalkyl, naphthyl or pyridyl group or a phenyl group optionally substituted by one or more substituents selected from halogen atoms and amino, alkyl, alkoxy and haloalkyl groups, and $R_2$ represents a phenyl group optionally substituted by one or more substituents selected from halogen atoms and nitro, alkyl, haloalkyl and haloalkylsulphonyl groups.

When an alkyl substituent is present this preferably contains from 1 to 12, especially 1 to 6, carbon atoms; and when a cycloalkyl substituent is present this preferably contains from 3 to 8, especially 5—8, carbon atoms.

Preferably $R_1$ represents an isopropyl, cyclohexyl, pyridyl, naphthyl or phenyl group, optionally substituted by a fluorine or chlorine atom, or an amino, methyl, isopropyl, methoxy or trifluoromethyl group. Although more than one such substituent may be present, it is preferred that a phenyl ring should be unsubstituted or monosubstituted.

Preferably $R_2$ represents a phenyl group optionally substituted by a chlorine or fluorine atom, a nitro group, a methyl or trifluoromethyl group, or a trifluoromethylsulhonyl group. It has been found that, in general, the highest levels of herbicidal activity are found in those compounds wherein $R_2$ is a phenyl group which is either unsubstituted or bears at least one substituent, preferably trifluoromethyl, at the 3-positon.

Many of the compounds of formula I are novel and the invention therefore also extends to these novel compounds *per se*. The novel compounds are the substituted tetrazoles of formula I wherein $R_1$ is as defined above and $R_2$ is a phenyl group substituted by a nitro, alkyl, haloalkyl, or haloalkylsulphonyl group with the proviso that if $R_1$ represents a methyl group, then $R_2$ does not represent a *m*-nitrophenyl group. Preferred novel compounds are those wherein $R_1$ is as defined above and $R_2$ is a phenyl group substituted at the 3-position, preferably by a trifluoromethyl group.

The invention also provides a process for the preparation of a novel compound of the general formula I, which comprises reacting a 5-halo tetrazole of formula II

II

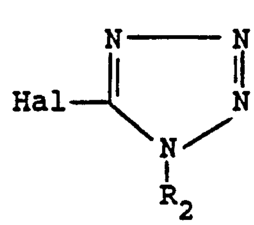

with an alcohol of formula $R_1OH$, wherein $R_1$ is as defined above, or an alkali metal salt of such an alcohol. The said 5-halo tetrazole may be prepared by (i) reacting an inorganic azide with an isocyano dihalide having the formula $R_2—N{=}C(Hal)_2$ wherein $R_2$ is as defined above and Hal represents a halogen, preferably chlorine, atom or (ii) reacting an aluminium azide with an isocyanate of formula $R_2NCO$ followed by treatment with phosphorus pentachloride.

The inorganic azide used in the tetrazole cyclisation step is suitably hydrazoic acid, ammonium azide, or an alkali or alkaline earth metal azide, in particular an alkali metal azide such as sodium azide. Both steps of the process are conveniently carried out in an inert solvent, which may be organic, such as acetone, or inorganic, e.g. water, or a mixture thereof.

The isocyanodihalide reactant may be prepared by known synthetic procedures, for example from the corresponding aniline of formula $R_2NH_2$ by reaction with formic acid followed by a mixture of sulphuryl chloride and thionyl chloride.

The compounds of the general formula I have useful herbicidal properties, and accordingly the invention provides also the use as herbicide of such a compound or a composition containing such a compound. Further, in accordance with the invention, there is provided a method of combating undesired plant growth at a locus by treating the locus with a compound or composition according to the invention. Application to the locus may be pre-emergence or post-emergence. The dosage of active ingredient used may, for example, be from 0.05 to 4 kg/ha. A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs magnesium aluminium slicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixturs of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% w of a dispersing agent and, where necessary, 0—10% w of stabilisers(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20 w/v of other additives such as stabilisers, penetrants and corrosion inhibitors. Suspension concentrates are usually compounded so as to obtain a stable, non-sedimenting flowable product and usually contain 10—75% w active ingredient, 0.5—15 w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is

substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or the oil-in-water type, and may have a thick 'mayonnaise'-like consistency.

The composition of the invention may also contain other ingredients, for example other compounds possessing herbicidal, insecticidal or fungicidal properties.

The following Examples illustrate the invention.

## Example 1

1-(3'-trifluoromethylphenyl)-5-phenoxy-1H tetrazole

A) Preparation of 3-formamidobenzotrifluoride

3-trifluoromethylaniline (80.5 g, 0.5 mole) and formic acid (230 g, 5 mole) were refluxed for 2 hours. The mixture was cooled to room temperature, and poured into ice/water. Product filtered, washed with water and dried to yield a solid, m.pt. 51—53°C.

B) Preparation of 3-trifluoromethylphenyl isocyanodichloride

3-formamidobenzotrifluoride (85.05 g, 0.45 mole) was added protionwise to a stirred mixture of sulphuryl chloride (60.75 g, 0.45 mole) and thionyl chloride (135 ml) at 20—25°C. The mixture was stirred 4 hours at room temperature then heated slowly to 80°C, hydrogen chloride and sulphur dioxide being liberated. The excess thionyl chloride was evaporated and a small amount of solid material removed by filtration. Product distilled under vacuum to yield a liquid, b.pt. 82—84°C/5 mm.

C) Preparation of 5-chloro-1-(3'-trifluoromethylphenyl)-1H-tetrazole

A solution of the isocyanodichoride prepared in B) (7.26 g, 0.03 mole) in acetone (25 ml) was added to a stirred solution of sodium azide (1.95 g; 0.03 mole) in water (10 ml). The temperature rose to about 50°C and the mixture was stirred at this temperature for 15 minutes, then heated to reflux for 30 minutes. The reaction mixture was cooled, poured into water and extracted with methylene chloride. The combined extracts were dried ($MgSO_4$) and evaporated to leave the crude product as a yellow oil. The oil was taken up in 40—60° petrol containing just enough ethyl acetate to effect complete solution and the product crystallised by cooling in a dry ice/acetone bath to yield a solid, m.pt. 44—46°C.

D) Preparation of 1-(3'-trifluoromethylphenyl)-5-phenoxy-1H-tetrazole

Anhydrous potassium carbonate (1.38 g, 0.01 mole) was added to a solution of the 5-chloro product obtained in C) (1.24 g, 0.005 mole) and phenol (0.47 g, 0.005 mole) in dry acetone (15 ml), and the mixture stirred at reflux overnight. The hot mixture was poured into ice/water, allowed to stand 1 hour and the product collected by filtration. Recrystallisation from ethanol/water yielded a solid, m.pt. 73—74°C.

| Analysis | | | |
|---|---|---|---|
| Calculated: | C 54.9 | H 2.96 | N 18.3% |
| Found: | C 54.8 | H 3.1 | N 18.4% |

## Example 2—33

Following procedures similar to those described in Example 1 (except as described below), a number of additional compounds were prepared, whose melting points and chemical analyses are shown in Table I below. In that Table, the compounds are identified by reference to the identity of the substituents in formula I.

In the case of Example 29, the 5-chloro tetrazole was prepared from p..tolylisocyanate by the following procedure.

A) A solution of aluminium chloride (13.3 g) in dry tetrahydrofuran (150 ml) was added to a suspension of sodium azide (17.6 g) in dry tetrahydrofuran (50 ml), containing p-tolylisocyanate (12 g). The reaction mixture was stirred under reflux for 24 hours, cooled in an ice/water bath and acidified with 20 ml 6N hydrochloric acid. The two phase mixture was then evaporated to dryness. The residue was digested with 3 × 100 ml portion of acetone, and after removal of the acetone the brown solid was dissolved in 250 ml hot ethanol, charcoal added, and filtered. Product crystallised by the addition of water to the filtrate.

B) Phosphorous pentachloride (10.4 g) was added to a stirred suspension of the product of A) (8.8 g) in dry toluene (100 ml). The mixture was heated to reflux and stirred at reflux for 4 hours. Chromatography on a silica gel column gave the desired product, 1-(4'-methylphenyl)-5-chloro-1H tetrazole, which was then reacted with phenol according to the procedure described in Example 1 D.

Table I

| Ex. No. | $R_1$ | $R_2$ | M.Pt. °C | Elemental Analysis | | | | | |
|---|---|---|---|---|---|---|---|---|---|
| | | | | Calculated | | | Found | | |
| | | | | C | H | N | C | H | N |
| 2 | 4-Cl-Phenyl | 3-$CF_3$-Phenyl | 107-108 | 49.3 | 2.37 | 16.4 | 49.5 | 2.5 | 16.7 |
| 3 | 2-Cl-Phenyl | " | 104 | 49.3 | 2.37 | 16.4 | 49.2 | 2.3 | 16.4 |
| 4 | 3-Cl-Phenyl | " | 82-83 | 49.3 | 2.37 | 16.4 | 49.4 | 2.3 | 16.6 |
| 5 | 2,4-$Cl_2$-Phenyl | " | 130 | 44.8 | 1.88 | 14.9 | 45.0 | 1.8 | 15.0 |
| 6 | 3,4-$Cl_2$-Phenyl | " | 107-108 | 44.8 | 1.88 | 14.9 | 45.1 | 1.7 | 18.0 |
| 7 | 3,5-$Cl_2$-Phenyl | " | 100-102 | 44.8 | 1.88 | 14.9 | 44.7 | 1.9 | 14.9 |
| 8 | 4-F-Phenyl | " | 75-77 | 51.9 | 2.49 | 17.3 | 51.5 | 2.1 | 17.6 |
| 9 | 3-F-Phenyl | " | 84-86 | 51.9 | 2.49 | 17.3 | 51.7 | 2.3 | 17.0 |
| 10 | 2-Cl-4-$CF_3$-Phenyl | " | 118-120 | 44.1 | 1.73 | 13.7 | 44.1 | 1.7 | 13.7 |
| 11 | 4-$CH_3$-Phenyl | " | 87-89 | 56.2 | 3.46 | 17.5 | 56.4 | 3.6 | 17.6 |
| 12 | 2-$CH_3$-Phenyl | " | 113-115 | 56.2 | 3.46 | 17.5 | 56.3 | 3.1 | 17.6 |
| 13 | 3-$CH_3$-Phenyl | " | 83-85 | 56.2 | 3.46 | 17.9 | 56.2 | 3.1 | 17.7 |
| 14 | 2,4-$(CH_3)_2$-Phenyl | " | 92-93 | 57.5 | 3.92 | 16.8 | 57.5 | 4.1 | 16.8 |

Table I continued...

| Ex. No. | $R_1$ | $R_2$ | M.Pt. °C | Elemental Analysis | | | | | |
|---|---|---|---|---|---|---|---|---|---|
| | | | | Calculated | | | Found | | |
| | | | | C | H | N | C | H | N |
| 15 | 2,6-$(CH_3)_2$-Phenyl | 3-$CF_3$-Phenyl | 125-127 | 57.5 | 3.92 | 16.8 | 57.1 | 4.1 | 16.7 |
| 16 | 4-iso$C_3H_7$-Phenyl | " | 65 | 58.6 | 4.34 | 16.1 | 58.7 | 4.6 | 16.1 |
| 17 | 3-Pyridyl | " | 87 | 50.8 | 2.62 | 22.8 | 50.8 | 2.6 | 22.7 |
| 18 | 4-Pyridyl | " | 137-139 | 50.8 | 2.62 | 22.8 | 50.7 | 2.6 | 22.7 |
| 19 | 3-$NH_2$-Phenyl | " | 85 | 52.3 | 3.14 | 21.8 | 52.4 | 3.1 | 21.7 |
| 20 | Cyclohexyl | " | 37-39 | 53.8 | 4.84 | 17.9 | 54.4 | 4.9 | 18.0 |
| 21 | 3-$CF_3$-Phenyl | " | 93-95 | 48.1 | 2.15 | 15.0 | 48.1 | 2.0 | 14.8 |
| 22 | 1-Naphthyl | " | 98-99 | 60.8 | 2.84 | 15.8 | 61.1 | 3.3 | 15.9 |
| 23 | 2-Naphthyl | " | 117 | 60.8 | 2.84 | 15.8 | 60.7 | 3.0 | 15.7 |
| 24 | 3-$CH_3O$-Phenyl | " | 82-83 | 53.6 | 3.3 | 16.7 | 52.9 | 3.3 | 16.4 |
| 25 | 4-$CH_3O$-Phenyl | " | 93-94 | 53.6 | 3.3 | 16.7 | 53.6 | 3.2 | 16.6 |
| 26 | Phenyl | Phenyl | 124-125 | 65.5 | 4.23 | 23.5 | 65.2 | 4.3 | 23.7 |
| 27 | " | 4-Cl-Phenyl | 115-116 | 57.3 | 3.33 | 20.5 | 56.5 | 3.3 | 20.5 |

Table I continued...

| Ex. No. | $R_1$ | $R_2$ | M.Pt. °C | Elemental Analysis Calculated C | Calculated H | Calculated N | Found C | Found H | Found N |
|---|---|---|---|---|---|---|---|---|---|
| 28 | " | 4-$NO_2$-Phenyl | 180-181 | 55.1 | 3.20 | 24.7 | 54.6 | 3.0 | 24.6 |
| 29 | " | 4-$CH_3$-Phenyl | 131-132 | 66.6 | 4.8 | 22.2 | 66.6 | 4.9 | 22.0 |
| 30 | " | 4-$CF_3$-Phenyl | 105-106 | 54.9 | 2.96 | 18.3 | 55.0 | 2.9 | 18.3 |
| 31 | " | 2-$CF_3$-Phenyl | 86 | 54.9 | 2.96 | 18.3 | 54.8 | 3.2 | 18.3 |
| 32 | " | 3,5-$(CF_3)_2$-Phenyl | 84-87 | 48.1 | 2.15 | 15.0 | 48.3 | 2.0 | 14.8 |
| 33 | 3-F-Phenyl | " | 74-75 | 45.9 | 1.8 | 14.3 | 45.9 | 1.8 | 14.2 |
| 34 | 4-Cl-Phenyl | Phenyl | 99-100 | | | | | | |
| 35 | 4-$CH_3$-Phenyl | Phenyl | 91-92 | 66.7 | 4.8 | 22.2 | 66.6 | 4.8 | 22.1 |
| 36 | 2-Cl-Phenyl | Phenyl | 78-79 | 57.2 | 3.3 | 20.5 | 57.5 | 3.4 | 20.6 |
| 37 | Phenyl | 3-$NO_2$-Phenyl | 115-117.5 | 55.2 | 3.2 | 24.7 | 55.0 | 3.1 | 24.7 |
| 38 | Phenyl | 3-$CF_3SO_2$-Phenyl | 118-119 | 45.4 | 2.4 | 15.1 | 45.4 | 2.4 | 15.2 |
| 39 | Phenyl | 2-Cl,3-$CF_3$-Phenyl | 85-88 | 49.4 | 2.4 | 16.4 | 49.1 | 2.3 | 16.2 |
| 40 | Phenyl | 4-Cl,3-$CF_3$-Phenyl | 119-121 | 49.4 | 2.4 | 16.4 | 49.4 | 2.1 | 16.3 |
| 41 | Phenyl | 4-$NO_2$,3-$CF_3$-Phenyl | 104-106 | 47.9 | 2.3 | 19.9 | 47.7 | 2.2 | 19.9 |
| 42 | Phenyl | 4-F,3-$CF_3$-Phenyl | 90 | 51.9 | 2.5 | 17.3 | 51.8 | 2.5 | 17.4 |
| 43 | i-$C_3H_7$ | 3-$CF_3$-Phenyl | oil | 48.5 | 4.1 | 20.6 | 48.6 | 4.3 | 20.8 |

Example 44

Herbicidal Activity

To evaluate their herbicidal activity, compounds according to the invention were tested using as a representative range of plants: maize, *Zea mays* (Mz); rice, *Oryza sativa* (R); barnyard grass, *Echinochloa crusgalli* (BG); oat, *Avena sativa* (O); linseed, *Linum usitatissisum* (L); mustard, *Sinapsis alba* (M); sugar beet, *Beta vulgaris* (SB) and soya bean, *Glycine max* (S).

The test falls into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horiticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray tests, and at a dosage level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed twelve days after spraying the foliage and the soil, and thirteen days after drenching the soil and were recorded on a 0—9 scale. A rating 0 indicates growth as untreated control, a rating 9 indicates death. An increase of 1 unit on the linear scale approximates to a 10% increase in the level of effect.

The results of the tests are set out in Table II below.

Table II

| Compound of Example Number | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 1 | 4 | 4 | 6 | 5 | 6 | 8 | 8 | 3 | 5 | 8 | 5 | 8 | 8 | 9 | 9 | 9 | 5 | 4 | 3 | 9 | 7 | 7 | 9 | 9 | 1 |
| | | | | | | | | | 1 | 3 | 2 | 7 | 6 | 8 | 8 | 8 | 4 | 3 | 2 | 9 | 6 | 5 | 7 | 9 | 1 |
| 2 | 0 | 0 | 0 | 0 | 0 | 0 | 0 | 0 | 5 | 0 | 0 | 2 | 0 | 4 | 7 | 3 | 0 | 0 | 0 | 0 | 0 | 0 | 0 | 0 | 0 |
| | | | | | | | | | 1 | 0 | 0 | 0 | 0 | 2 | 5 | 3 | 0 | 0 | 0 | 0 | 0 | 0 | 0 | 0 | 0 |
| 4 | 0 | 0 | 0 | 0 | 0 | 0 | 0 | 0 | 5 | 2 | 0 | 2 | 3 | 8 | 9 | 4 | 3 | 0 | 0 | 6 | 5 | 2 | 4 | 6 | 0 |
| | | | | | | | | | 1 | 0 | 0 | 0 | 1 | 6 | 8 | 2 | 2 | 0 | 0 | 5 | 3 | 0 | 1 | 4 | 0 |
| 13 | 0 | 0 | 0 | 0 | 0 | 0 | 0 | 0 | 5 | 4 | 3 | 3 | 4 | 4 | 4 | 6 | 6 | 0 | 2 | 8 | 4 | 3 | 4 | 8 | 0 |
| | | | | | | | | | 1 | 3 | 3 | 3 | 3 | 2 | 2 | 3 | 4 | 0 | 0 | 6 | 4 | 3 | 0 | 5 | 0 |
| 8 | 0 | 0 | 0 | 0 | 0 | 0 | 0 | 0 | 5 | 6 | 4 | 2 | 5 | 7 | 8 | 8 | 4 | 0 | 0 | 8 | 6 | 6 | 8 | 9 | 6 |
| | | | | | | | | | 1 | 2 | 2 | 2 | 2 | 5 | 5 | 6 | 4 | 0 | 0 | 6 | 2 | 3 | 4 | 9 | 2 |
| 14 | 0 | 0 | 0 | 0 | 0 | 0 | 0 | 0 | 5 | 0 | 0 | 0 | 0 | 2 | 4 | 2 | 3 | 0 | 0 | 0 | 0 | 0 | 0 | 0 | 0 |
| | | | | | | | | | 1 | 0 | 0 | 0 | 0 | 2 | 3 | 2 | 2 | 0 | 0 | 0 | 0 | 0 | 0 | 0 | 0 |

Table II continued

| Compound of Example Number | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 16 | 0 | 0 | 0 | 0 | 0 | 0 | 0 | 0 | 5 | 2 | 3 | 3 | 4 | 5 | 5 | 3 | 2 | 0 | 0 | 0 | 0 | 0 | 0 | 0 | 0 |
| | | | | | | | | | 1 | 2 | 2 | 2 | 2 | 2 | 5 | 3 | 2 | 0 | 0 | 0 | 0 | 0 | 0 | 0 | 0 |
| 17 | 0 | 0 | 0 | 0 | 0 | 0 | 0 | 0 | 5 | 5 | 4 | 4 | 4 | 6 | 9 | 4 | 4 | 0 | 0 | 0 | 0 | 0 | 0 | 0 | 0 |
| | | | | | | | | | 1 | 2 | 3 | 2 | 3 | 2 | 4 | 4 | 3 | 0 | 0 | 0 | 0 | 0 | 0 | 0 | 0 |
| 20 | 0 | 0 | 0 | 0 | 0 | 0 | 0 | 0 | 5 | 3 | 0 | 7 | 4 | 5 | 6 | 5 | 6 | 1 | 1 | 7 | 5 | 2 | 5 | 6 | 2 |
| | | | | | | | | | 1 | 2 | 0 | 4 | 2 | 4 | 4 | 3 | 3 | 0 | 0 | 3 | 3 | 0 | 3 | 3 | 0 |
| 9 | 0 | 0 | 4 | 0 | 2 | 0 | 2 | 0 | 5 | 3 | 0 | 8 | 6 | 8 | 9 | 8 | 3 | 0 | 0 | 6 | 5 | 6 | 6 | 9 | 0 |
| | | | | | | | | | 1 | 0 | 0 | 5 | 3 | 5 | 7 | 7 | 3 | 0 | 0 | 5 | 3 | 4 | 2 | 7 | 0 |
| 26 | N/T | | | | | | | | 5 | – | – | – | – | – | – | – | – | N/T | | | | | | | |
| | | | | | | | | | 1 | 2 | 0 | 0 | 3 | 4 | 4 | 3 | 4 | | | | | | | | |

Table II continued

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 38 | 0 | 0 | 0 | 0 | 0 | 0 | 0 | 0 | 5 | 4 | 3 | 4 | 2 | 4 | 4 | 4 | 3 | 0 | 0 | 0 | 0 | 0 | 0 | 0 | 0 |
| | | | | | | | | | 1 | 1 | 2 | 2 | 1 | 2 | 3 | 1 | 2 | 0 | 0 | 0 | 0 | 0 | 0 | 0 | 0 |
| 39 | 0 | 0 | 0 | 0 | 0 | 0 | 0 | 0 | 5 | 5 | 2 | 2 | 0 | 4 | 7 | 8 | 6 | 0 | 0 | 0 | 0 | 0 | 0 | 0 | 0 |
| | | | | | | | | | 1 | 4 | 2 | 2 | 0 | 2 | 5 | 5 | 5 | 0 | 0 | 0 | 0 | 0 | 0 | 0 | 0 |
| 41 | 0 | 0 | 0 | 0 | 4 | 4 | 4 | 2 | 5 | 5 | 2 | 4 | 0 | 0 | 3 | 2 | 4 | 0 | 0 | 0 | 0 | 0 | 0 | 0 | 0 |
| | | | | | | | | | 1 | 2 | 1 | 2 | 0 | 0 | 2 | 1 | 2 | 0 | 0 | 0 | 0 | 0 | 0 | 0 | 0 |
| 43 | 3 | 2 | 4 | 3 | 2 | 3 | 4 | 0 | 5 | 3 | 2 | 3 | 4 | 4 | 0 | 3 | 4 | 4 | 2 | 8 | 4 | 3 | 4 | 8 | 4 |
| | | | | | | | | | 1 | 0 | 0 | 0 | 0 | 3 | 0 | 0 | 3 | 0 | 0 | 3 | 2 | 0 | 0 | 3 | 2 |

## Claims

1. A herbicidal composition which comprises at least one carrier which is a surface active agent, and, as active ingredient, a substituted tetrazole of the formula:—

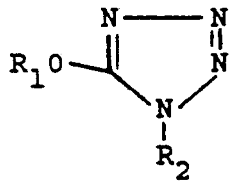

I

wherein $R_1$ represents an alkyl, cycloalkyl, naphthyl or pyridyl group or a phenyl group optionally substituted by one or more substituents selected from halogen atoms and amino, alkyl, alkoxy and haloalkyl groups, and $R_2$ represents a phenyl group optionally substituted by one or more substituents selected from halogen atoms and nitro, alkyl, haloalkyl and haloalkylsulphonyl groups.

2. A composition as claimed in claim 1, in which the active ingredient is tetrazole of formula I wherein $R_1$ represents an isopropyl, cyclohexyl, pyridyl or napthyl group or a phenyl group optionally substituted by a fluorine or chlorine atom or an amino, methyl, isopropyl, methoxy or trifluoromethyl group.

3. A composition as claimed in claim 2, wherein $R_1$ represents an unsubstituted or mono-substituted phenyl group.

4. A composition as claimed in any of claims 1 to 3, wherein $R_2$ represents a phenyl group optionally substituted at the 3-position by a trifluoromethyl group.

5. A substituted tetrazole of the formula I as shown in claim 1, wherein $R_1$ is as defined in claim 1 and $R_2$ represents a phenyl group substituted by a nitro, alkyl, haloalkyl, or haloalkylsulphonyl group, provided that if $R_1$ represents a methyl group, $R_2$ does not represent a *m*-nitrophenyl group.

6. A substituted tetrazole as claimed in claim 5, wherein $R_1$ represents a phenyl group optionally mono-substituted by a fluorine or chlorine atom or an amino, methyl, isopropyl, methoxy or trifluoromethyl group.

7. A substituted tetrazole as claimed in claim 5 or 6, wherein $R_2$ represents a phenyl group substituted at the 3-position by a trifluoromethyl group.

8. A process for the preparation of a compound as claimed in claim 5, which comprises reacting a 5-halotetrazole of formula II

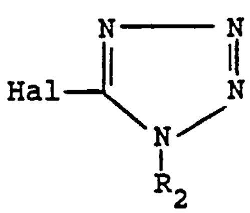

II

with an alcohol of formula $R_1OH$, wherein $R_1$ and $R_2$ are as defined in claim 5, or an alkali metal salt of such an alcohol.

9. Process as claimed in claim 8, wherein the 5-halo tetrazole of formula II is prepared by reacting (i) an isocyanate of formula $R_2NCO$ with aluminium azide followed by phosphorus pentachloride, or (ii) an isocyano dihalide of formula $R_2N{=}C(Hal)_2$ with an inorganic azide; $R_2$ being as defined in claim 6 and Hal representing a halogen atom.

10. Method of combating undesired growth at a locus, which comprises treating the locus with a composition as claimed in any one of claims 1 to 4 or a compound as claimed in any one of claims 5 to 7.

11. Use as a herbicide of a substituted tetrazole as defined in any one of claims 1 to 7.

## Patentansprüche

1. Herbizide Zusammensetzung, die wenigstens einen Träger, der ein oberflächenaktives Mittel ist, und als wirksamen Bestandteil ein substituiertes Tetrazol der Formel:

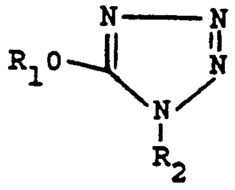

I

umfaßt, worin

$R_1$ eine Alkyl-, Cycloalkyl-, Naphthyl- oder Pyridylgruppe oder eine gegebenenfalls durch einen oder mehrere Substituenten, ausgewählt unter Halogenatomen und Amino-, Alkyl-, Alkoxy- und Halogenalkylgruppen, substituierte Phenylgruppe bedeutet und

$R_2$ eine Phenylgruppe darstellt, die gegebenenfalls durch einen oder mehrere Substituenten, ausgewählt unter Halogenatomen und Nitro-, Alkyl-, Halogenalkyl- und Halogenalkylsulfonylgruppen, substituiert ist.

2. Zusammensetzung nach Anspruch 1, worin der wirksame Bestandteil ein Tetrazol der Formel I ist, worin $R_1$ eine Isoprpyl-, Cyclohexyl-, Pyridyl- oder Naphthylgruppe oder eine Phenylgruppe bedeutet, die gegebenenfalls durch ein Fluor- oder Chloratom oder eine Amino-, Methyl-, Isopropyl-, Methoxy- oder Trifluormethylgruppe substituiert ist.

3. Zusammensetzung nach Anspruch 2, worin $R_1$ eine unsubstituierte oder mono-substituierte Phenylgruppe darstellt.

4. Zusammensetzung nach einem der Ansprüche 1 bis 3, worin $R_2$ eine Phenylgruppe darstellt, die gegebenenfalls in der 3-Stellung durch eine Trifluormethylgruppe substituiert ist.

5. Ein substituiertes Tetrazol der Formel I, wie in Anspruch 1 dargestellt, worin $R_1$ wie in Anspruch 1 definiert ist und $R_2$ eine durch eine Nitro-, Alkyl-, Halogenalkyl- oder Halogenalkylsulfonylgruppe substituierte Phenylgruppe darstellt, mit der Maßgabe, daß dann, wenn $R_1$ ine Methylgruppe bedeutet, $R_2$ nicht eine *m*-Nitrophenylgruppe darstellt.

6. Substituiertes Tetrazol nach Anspruch 5, worin $R_1$ eine gegebenenfalls durch ein Fluor- oder Chloratom oder eine Amino-, Methyl-, Isopropyl-, Methoxy- oder Trifluormethylgruppe mono-substituierte Phenylgruppe bedeutet.

7. Substituiertes Tetrazol nach Anspruch 5 oder 6, worin $R_2$ eine in der 3-Stellung durch eine Trifluormethylgruppe substituierte Phenylgruppe bedeutet.

8. Verfahren zur Herstellung einer Verbindung nach Anspruch 5, welches ein Umsetzen eines 5-Halogentetrazols der Formel II

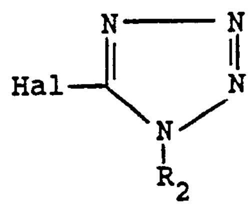

II

mit einem Alkohol der Formel $R_1OH$, worin $R_1$ und $R_2$ wie in Anspruch 5 definiert sind, oder mit einem Alkalimetallsalz eines solchen Alkohols umfaßt.

9. Verfahren nach Anspruch 8, worin das 5-Halogentetrazol der Formel II durch Umsetzen

(i) eines Isocyanats der Formel $R_2NCO$ mit Aluminiumazid und anschließend mit Phosphorpentachlorid oder

(ii) eines Isocyanodihalogenids der Formel $R_2N{=}(Hal)_2$ mit einem anorganischen Azid hegestellt wird, wobei $R_2$ wie in Anspruch 6 definiert ist und Hal ein Halogenatom bedeutet.

10. Verfahren zur Bekämpfung von unerwünschtem Wachstum an einem Ort, welches ein Behandeln des Ortes mit einer Zusammensetzung, wie in einem der Ansprüche 1 bis 4 beansprucht, oder mit einer Verbindung, wie in einem der Ansprüche 5 bis 7 beansprucht, umfaßt.

11. Verwendung eines substituierten Tetrazols, wie in einem der Ansprüche 1 bis 7 definiert, als ein Herbizid.

**Revendications**

1. Une compositions herbicide qui comprend au moins un véhicule qui est un agent tensio-actif et, comme ingrédient actif, un tétrazole substitué de la formule:

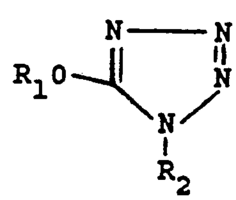

I

où $R_1$ représente un groupe alcoyle, cycloalcoyle, naphtyle ou pyridyle ou un groupe phényle éventuellement substitué par un ou plusieurs substituants choisis parmi des atomes d'halogènes et des groupes amino, alcoyle, alcoxy et halocoyle, et $R_2$ représente un groupe phényle éventuellement substitué par un ou plusieurs substituants choisis parmi des atomes d'halogènes et des groupes nitro, alcoyle, haloalcoyle et haloalcoylsulfonyle.

2. Une composition selon la revendication 1, dans laquelle l'ingrédient actif est un tétrazole de formule I où $R_1$ représente un groupe isoproyle, cyclohexyle, pyridyle ou naphtyle ou un groupe phényle éventuellement substitué par par un atome de fluor ou de chlore ou un groupe amino, métyle, isopropyle, méthoxy ou trifluorométhyle.

3. Une composition selon la revendication 2, dans laquelle $R_1$ représente un groupe phényle non-substitué ou monosubstitué.

4. Une composition selon l'une quelconque des revendications 1 à 3, dans laquelle $R_2$ représente un groupe phényle éventuellement substitué à la position 3 par un groupe trifluorométhyle.

5. Un tétrazole substitué de la formule I telle que représenté dans la revendication 1, où $R_1$ est tel que défini dans la revendication 1 et $R_2$ représente un groupe phényle substitué par un groupe nitro, alcoyle, haloalcoyle ou haloalcoylsulfonyle, avec la condition que si $R_1$ représente un groupe méthyle, alors $R_2$ ne représente pas un groupe *m*-nitrophényle.

6. Un tétrazole substitué selon la revendication 5, dans lequel $R_1$ représente un groupe phényle éventuellement monosubstitué par un atome de fluor ou de chlore ou par un groupe amino, méthyle, isopropyle, méthoxy ou trifluorométhyle.

7. Un tétrazole substitué selon la revendication 5 ou 6, dans lequel $R_2$ représente un groupe phényle substitué à la position 3 par un groupe trifluorométhyle.

8. Un procédé pour la préparation d'un composé tel que revendiqué dans la revendication 5, qui comprend la réaction d'un 5-halotétrazole de formule II

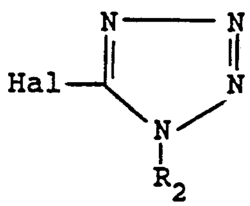

II

avec un alcool de formule $R_1OH$, où $R_1$ et $R_2$ sont tels que définis dans la revendication 5, ou avec un sel de métal alcalin d'un tel alcool.

9. Une procédé selon la revendication 8, dans lequel le 5-halotétrazole de formule II est préparé en faisant réagir (1) un isocyanate de formule $R_2NCO$ avec l'azoture d'aluminium et ensuite avec le pentaclorure de phosphore ou (2) un isocyano dihalogénure de formule $R_2N{=}C(Hal)_2$ avec un azoture inorganique; $R_2$ étant tel que défini dans la revendication 6 et Hal représentant un atome d'halogène.

10. Méthode de lutte contre la croissance de végétation indésirable en un lieu, qui comprend le traitement du lieu avec une composition selon l'une quelconque des revendications 1 à 4 ou avec un composé selon l'une quelconque des revendications 5 à 7.

11. Utilisation comme herbicide d'un tétrazole substitué tel que défini dans l'une quelconque des revendications 1 à 7.